# EUROPÄISCHE PATENTSCHRIFT

(11) **EP 2 316 350 B1**
(45) Veröffentlichungstag und Bekanntmachung des Hinweises auf die Patenterteilung: **07.05.2014**
(21) Anmeldenummer: 10188210.8
(22) Anmeldetag: 20.10.2010
(51) Int. Cl.: A61B 17/10, A61B 17/128, A61B 18/14, A61B 17/068, A61B 17/122, A61B 18/00, A61B 17/00, A61B 17/32

(54) **Resektionsvorrichtung**
Resection device
Dispositif de résection

(30) Priorität: 27.10.2009 DE 102009050829
(43) Veröffentlichungstag der Anmeldung: 04.05.2011
(73) Patentinhaber: Ovesco Endoscopy AG, 72074 Tübingen (DE)
(72) Erfinder: Baur, Franziska, 73265, Dettingen unter Teck (DE); Ho, Chi-Nghia, 72762 Reutlingen (DE); Schurr, Marc O., 72072, Tübingen (DE); Anhöck, Gunnar, 72764, Reutlingen (DE); Gottwald, Thomas, 82431, Kochel am See (DE)
(74) Vertreter: Winter, Brandl, Fürniss, Hübner, Röss, Kaiser, Polte - Partnerschaft

(56) Entgegenhaltungen:
- WO-A1-99/25255
- DE-A1- 10 356 018
- DE-U1-202008 007 774
- US-A1- 2002 062 130
- US-A1- 2004 158 127
- US-B1- 6 306 081

## Beschreibung

Die vorliegende Erfindung betrifft eine Resektionsvorrichtung zur minimalinvasiven (Vollwand-) Resektion eines Gewebes beispielsweise einer Magenwand oder eines Kolon.

Eine derartige Vorrichtung verwendet ein endoskopisches Zuführmittel für einen Gewebeclip zum Verschließen des Gewebeschnitts und einer über das endoskopische Zuführmittel zuführbaren Schneidvorrichtung.

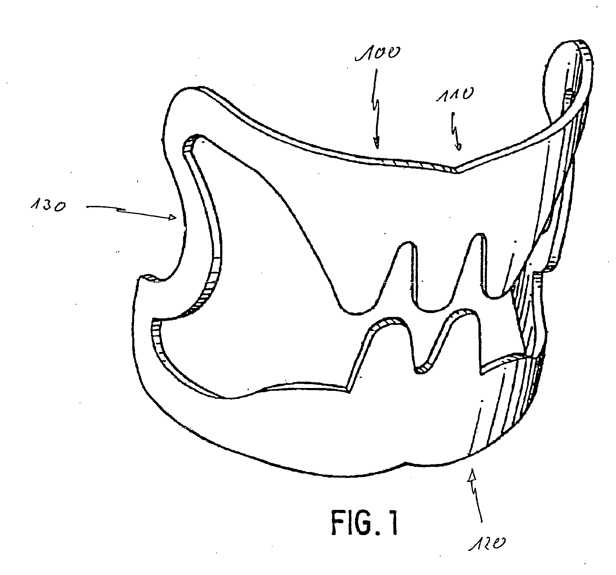

Aus dem Stand der Technik beispielsweise gemäß der US 6,849,078 B2 ist ein Gewebeclip dieser Gattung hinsichtlich seiner grundsätzlichen Konstruktion allgemein bekannt. Zum besseren Verständnis wird dieser Clip nachstehend unter Verweis auf die Fig. 1 näher beschrieben.

Demzufolge besteht ein solcher Clip 100 aus einer maulartigen Klemmeinrichtung mit zwei gezahnten Kiefern 110, 120, welche über zwei seitliche Scharniere 130 bzw. über flexible Ausformungen auf- und zugeklappt werden können. Die Scharniere 130 bzw. die flexiblen Ausformungen sind dabei vorzugsweise aus federelastischen Bändern ausgebildet, welche beim Aufklappen der Kiefer 110, 120 eine Federenergie speichern, die beim Freigeben der Kiefer 110, 120, d.h. bei einem Auslösen der Scharniere 130 bzw. der flexiblen Ausformungen zu einem Zuschnappen der Kiefer 110, 120 mit vorbestimmter Klemmkraft führt.

Im Einzelnen ist jeder Clip 100 einstückig aus einem Federblech gestanzt oder gelasert, indem aus dem Federblech ein Ring mit partiell unterschiedlicher Ringbreite herausgearbeitet wird. Zwei diametrisch gegenüberliegende Ringabschnitte mit großer Ringbreite bilden die beiden Kiefer 110, 120, wohingegen die beiden dazwischen liegenden Ringabschnitte mit schmaler Ringbreite die Scharniere 130 bzw. die flexiblen (elastischen) Ausformungen ergeben. Die Kiefer 110, 120 sind dadurch ausgebildet, indem die Ringabschnitte mit großer Ringbreite über deren Flachseite bogenförmig gewölbt werden, wohingegen die beiden Ringabschnitte mit schmaler Ringbreite um ihre Längsachse um ca. 180° verdreht (tordiert) ausgebildet sind, um die Scharniere auszuformen. Durch diese besondere Formgebung des gelaserten Federblechs entsteht die Form einer Art Haifischmaul mit zwei aufeinander sich zu bewegenden Zahnreihen, welche durch Lasern der Ringabschnitte mit großer Ringbreite gebildet werden.

Die Funktionsweise des vorstehend beschriebenen medizinischen Gewebeclips 100 lässt sich wie Folgt beschreiben:
Im Allgemeinen stellt eine endoskopische Implantation einer medizinischen Vorrichtung insgesamt das für den Patienten verträglichste Verfahren dar. In diesem Fall muss die medizinische Vorrichtung von der Innenseite eines Hohlorgans an diesem fixiert werden. Zu diesem Zwecke werden eine Anzahl (mindestens eine) der vorstehend beschriebenen Gewebeklammern, Clips oder Anker mittels eines Endoskops oder eines ähnlichen Zuführmittels in das Hohlorgan eingeführt und an vorbestimmten Stellen an der Organinnenseite platziert. Hiefür wird der jeweilige Clip oder Anker an das Organgewebe herangeführt und die Vorspannfeder für ein Zuschnappen des Clips oder Aufspannen des Ankers ausgelöst. Dieser hält oder klemmt daraufhin eine Gewebefalte zwischen seinen Kiefern oder seinen Haken oder Nadeln mit einer vorbestimmten Klemm- oder Spreizkraft ein, wobei sich die Zähne, Haken, Nadeln oder Zacken jedes Kiefers in das Gewebe bohren und dieses vorzugsweise durchdringen.

Auf diese Weise wird jeder Clip oder Anker in bestimmten Abständen zueinander an der Organinnenseite verankert.

Das in der Fig. 1 nicht weiter dargestellte Endoskop oder dergleichen Zuführmittel ist in der Regel mit einem Endoskopkopf oder einer Endoskopkappe ausgerüstet, welche neben den für ein Endoskop generell erforderlichen Funktionen wie Beleuchtung, Optik und ggf. Spülvorrichtung zusätzlich eine Halte- und Abzieheinrichtung für den Gewebeclip aufweist. An dieser Stelle sei darauf hingewiesen, dass in dieser gesamten Anmeldung unter einem Endoskop auch eine einfache Einführhilfe ohne eigene Beleuchtung und Optik sowie Spülfunktion verstanden werden kann.

Die Halte- und Abzieheinrichtung besteht im Wesentlichen aus einer Spreizhülse sowie einem manuell oder ferngesteuert betätigbaren Schieber, der in Endoskoplängsrichtung bewegbar ist. Die Spreizhülse ist dabei derart ausgebildet, dass der bereits geöffnete Gewebeclip auf die Hülse aufgesetzt werden kann, derart, dass ein nach hinten Rutschen des Clips während dessen Einführung in das Hohlorgan verhinderbar ist. Zu diesem Zweck ist der Schieber axial hinter dem Clip positioniert und dient quasi als Axialanschlag für den Clip.

Sobald der Clip an einer bestimmten Stelle platziert werden soll, wird der Schieber axial nach vorne bewegt und streift dabei den Clip über die Spreizhülse ab. Dabei wird der Clip ausgelöst, d.h. der vorstehend anhand der Fig. 1 beschriebene Vorspannmechanismus innerhalb des Clips wird beim Abstreifen von der Spreizhülse freigegeben und die beiden Kiefer des Gewebeclips schnappen unter Einklemmen des dazwischen liegenden Gewebes zu.

Aus einem weiteren Stand der Technik beispielsweise gemäß der DE 10/2004 037 830 A1 ist eine Endoskopkappe bekannt, die mit einer Schneidevorrichtung ausgerüstet ist. Die Endoskopkappe ist zu einem becher- oder napfförmigen Körper ausgebildet, der auf das distale Ende eines Endoskopschafts aufgesteckt ist. Der äußerste distale Rand des Bechers ist radial nach Innen abgekantet und bildet so eine innere Anlagefläche, an der die Schneidevorrichtung in form einer bestrombaren Kabelschlinge anliegt. Die Kabelschlinge wird dabei über nasenförmige, flexible Vorsprünge auf der Innenseite der Becherwand an der Anlagefläche gehalten. Die Kabelschlinge ist ferner über ein Stromzuführkabel mit der Außenseite verbunden, welches durch einen Endoskopschaft verschiebbar geführt ist

Um Gewebe zu entfernen, wird der Becher am distalen Ende des Endoskops auf dem Gewebe aufgesetzt und das Gewebe in den Becher eingesaugt. Anschließend wird die Kabelschlinge über das Stromzuführkabel zugezogen, wodurch das eingesaugte Gewebe eingeschnürt wird. Durch Bestromen der Schlinge wird das abgeschnürte Gewebe abgetrennt.

Mit einer derartigen Vorrichtung ist es zwar möglich, die Oberfläche beispielsweise einer Darm- oder Magenwand abzutrennen. Oftmals ist dieser Eingriff zu wenig radikal, um auch tiefer liegendes erkranktes Gewebe zu entfernen. Insbesondere ist an dieser Stelle darauf zu verweisen, dass bei einem solchen Eingriff das grundsätzliche Risiko eines Wanddurchbruchs besteht, was zu besonders nachteiligen Folgen für einen Patienten führt.

Aus dem Stand der Technik beispielsweise gemäß der US 2004/0158127 A1 sowie der DE 10 2004 037 830 A1 ist ein Instrument bekannt, das eine Schleimhaut in eine Kappe an einem distalen Ende eines Endoskops ansaugt und einen dadurch entstehenden ausgebauchten Teil unter Verwendung einer Diathermieschlinge abträgt. Aus US 6 306 081 B1 ist eine abnehmbare Kappe für ein Endoskop mit einem dehnbaren Ballon an dessen distalen Ende, um eine Beeinträchtigung eines Sichtfeldes und damit einer Behandlungsmöglichkeit zu verhindern, offenbart.

Ferner ist aus WO 99/25255 eine an ein Endoskop koppelbare Vorrichtung zum Abtrennen von Läsionen bekannt, wobei ein Benutzer die Wahl zwischen einer Ligatorbandligation, einer Abtrennschlingenligation oder einer Kombination beider an einer ins Auge gefassten Läsion hat.

Außerdem ist aus den Druckschriften DE 20 2008 007 774 U1 und US 2002/0062130 A1 jeweils eine Endoskopkappe mit Halte- und Abzieheinrichtung für einen Gewebeclip bekannt, der auf eine Spreizhülse der Endoskopkappe aufschiebbar ist. DE 20 2008 007 774 U1 bildet die Basis für den Oberbegriff des Anspruchs 1.

Angesichts dieses Stands der Technik besteht die Aufgabe der vorliegenden Erfindung darin, die bekannte Schneidevorrichtung derart weiterzubilden, dass eine Gewebeabtrennung mit geringerem Risiko für den Patienten möglich ist.

Diese Aufgabe wird durch eine Resektionsvorrichtung gelöst, welche die technischen Merkmale gemäß dem Patentanspruch 1 aufweist.

Demzufolge hat die erfindungsgemäße Resektionsvorrichtung eine becher- oder napfförmige Kappe für ein schaftartiges Einführmittel, die am distalen Ende des schaftartigen Einführmittels befestigbar oder daran ausgebildet ist und die einen Spreizhülsenabschnitt hat, auf den ein Gewebeclip der vorstehend genannten Bauart aufsteckbar ist, der mittels einer Auslöse- oder Abziehvorrichtung über die distale Stirnkante der Kappe abziehbar ist. Innerhalb des Spreizhülsenabschnitts ist eine Schneidevorrichtung angeordnet, die vorzugsweise über ein an der Innenwand des Spreizhülsenabschnitts angeordnetes oder ausgebildetes Distanzstück in einem vorbestimmten axialen Abstand zur distalen Stirnkante der Kappe gehalten ist, sodass eine Berührung zwischen Gewebeclip und Schneidevorrichtung vermieden wird.

Dabei ist die Schneidvorrichtung in Form einer Kabelschlinge ausgebildet, wofür an der Innenwand des Spreizhülsenabschnitts ein Distanzstück vorzugsweise in Form eines Rings angeordnet oder einstückig mit der Kappe derart ausgebildet ist, dass sich am distalen Endbereich des Spreizhülsenabschnitts eine innere Umfangsnut ausbildet, die entsprechend dem Distanzstück axial von der distalen Stirnseite des Spreizhülsenabschnitts beabstandet ist und eine Klemmfläche für die Kabelschlinge bildet.

Hierdurch ist es möglich, Gewebe in die Kappe einzuziehen und/oder zu saugen, um dann den Gewebeclip auszulösen, der das Gewebe unmittelbar vor der distalen Stirnkante der Kappe bzw. des Spreizhülsenabschnitts einklemmt. Wird daraufhin die Schneidevorrichtung aktiviert, kann das eingezogene Gewebe abgetrennt werden, wobei die Abtrennlinie in einem bestimmten Abstand zum Gewebeclip gezogen ist. Dieser Abstand wird vorzugsweise durch das Distanzstück bewirkt und ist so eingestellt, dass zum Einen ein in Kontakt kommen der Schneidevorrichtung mit dem Clip sicher vermieden wird und/oder zum Anderen der Clip noch ausreichend Gewebe klemmt, um den Schnitt sicher und dicht zu verschließen bzw. verschlossen zu halten (ohne Gefahr eines Ausreissens). Alternativ hierzu kann die Schneidevorrichtung beispielsweise auch durch eine lösbare Halterung oder Klebung an der Innenwand der Spreizhülse in dem vorbestimmten Abstand zur distalen Stirnkante fixiert sein, was jedoch nicht Teil der Erfindung ist.

Weiter vorzugsweise ist eine in Umfangsrichtung des Spreizhülsenabschnitts beidseitig geöffnete Stirnnut am Außenumfang der Spreizhülse zur Aufnahme des Gewebeclips ausgebildet, durch die vorzugsweise ein Faden, Kabel oder Gewebe als Auslöse- oder Abziehvorrichtung in Radialrichtung gezogen ist. Der Faden ist an einem Ende am schaftartigen Einführmittel bzw. an der Kappe fixiert und am anderen Ende längs des schaftartigen Einführmittels bewegbar geführt, sodass der Faden beim Einschieben des Gewebeclips in die Nut von diesem mitgenommen werden kann. Wird der Faden anschließend gezogen, zeigt dieser die Tendenz, sich innerhalb der Stirnnut zu verkürzen, wobei der Gewebeclip vom Faden wieder aus der Nut geschoben wird.

Alternativ hierzu kann der Faden oder das Kabel oder Gewebe an seinem einen Ende mit einem Abstreifring befestigt sein, der auf der Spreizhülse hinter dem aufgebogenen Clip axialverschieblich sitzt und bei Ziehen des Fadens nach vorn in Richtung distale Stirnseite der Kappe verschoben wird. Dabei wir der Clip über die distale Stirnkante abgezogen. Im Fall eines Abstreifrings kann auf die Anordnung einer Axialnut verzichtet werden.

Hierdurch wird folglich eine funktionsbezogene konstruktive Aufteilung der erfindungsgemäßen Halte- und Abzieheinrichtung erreicht, nämlich in die Anordnung der einen Axialanschlag ausbildenden Stirnnut/ -schlitz sowie in die hierzu separate Anordnung eines einen Schieber simulierenden Fadens oder den vom Faden betätigten Schiebering selbst. Diese konstruktive Zweiteilung der Halte- und Abzieheinrichtung ermöglicht es, insbesondere die Abzieheinrichtung als hochflexiblen Faden oder Kabel auszubilden, der nur wenig Bauraum benötigt und trotzdem ausreichend große Verschiebekräfte insbesondere bei Anwendung des vorstehend beschriebenen Flaschenzugmechanismus auf den Clip ausüben kann.

Die Erfindung sieht vor, die Schneidevorrichtung als eine Kabelschlinge auszubilden, die über ein durch das Zuführmittel hindurch geführtes oder längs des Zuführmittels geführtes Stromkabel betätigbar und bestrombar ist.

Die Spreizhülse ist an ihrem distalen Endabschnitt mit einem inneren Absatz oder einer Umfangsnut auszubilden, der/die axial von der distalen Stirnseite der Hülse beabstandet ist und eine Klemmfläche für die Kabelschlinge bildet.

In ersten Fall ist das Distanzstück in Form eines Rings als ein zur Spreizhülse separates Bauteil gefertigt, das in die Hülse eingesteckt wird und die Schlinge zwischen sich und dem Absatz einklemmt. Im zweiten Fall kann das Distanzstück auch einstückig mit der Spreizhülse gefertigt sein, wobei die Schlinge in die Umfangsnut eingelegt oder eingedrückt wird.

Vorzugsweise hat das Distanzstück eine axiale Länge von 2 bis 6 mm und insbesondere 2 bis 3.5 mm und schließt im Wesentlichen bündig mit der distalen Stirnkante der Spreizhülse ab.

Weitere vorteilhafte Ausgestaltungen der Erfindung sind Gegenstand der Unteransprüche.

Die Erfindung wird nachstehend anhand bevorzugter Ausführungsbeispiele unter Bezugnahme auf die begleitenden Figuren näher erläutert.

Es zeigen
Fig. 1 die beispielhafte Konstruktion eines Gewebeclips, wie er bereits aus dem Stand der Technik bekannt ist und wie er bei der vorliegenden Erfindung ebenfalls verwendet wird,
Fig. 2 den Längsschnitt einer Endoskopkappe für eine erfindungsgemäße Schneidevorrichtung gemäß einem ersten bevorzugten Ausführungsbeispiel der Erfindung mit aufgesetztem Gewebeclip gemäß Fig. 1 in schematischer Darstellung,
Fig. 3 den Längsschnitt einer Endoskopkappe für eine erfindungsgemäße Schneidevorrichtung gemäß einem zweiten bevorzugten Ausführungsbeispiel der Erfindung mit aufgesetztem schematisch dargestellten Gewebeclip,
Fig. 4 den Längsschnitt einer Endoskopkappe für eine erfindungsgemäße Schneidevorrichtung gemäß einem dritten bevorzugten Ausführungsbeispiel der Erfindung mit aufgesetztem Gewebeclip,
Fig. 5 den Längsschnitt einer Endoskopkappe für eine erfindungsgemäße Schneidevorrichtung gemäß einem vierten bevorzugten Ausführungsbeispiel der Erfindung mit aufgesetztem Gewebeclip,
Fig. 6 die Perspektivenansicht einer Endoskopkappe für eine erfindungsgemäße Schneidevorrichtung gemäß einem fünften bevorzugten Ausführungsbeispiel der Erfindung mit einer ringförmigen Abwurfeinrichtung für den Gewebeclip in unbetätigtem Zustand,
Fig. 7 die Perspektivenansicht der Endoskopkappe von Fig. 6 mit der ringförmigen Abwurfeinrichtung für den Gewebeclip in betätigtem Zustand und
Fig. 8 die Längsdarstellung der Endoskopkappe von Fig. 5 in unbetätigtem und betätigtem Zustand.

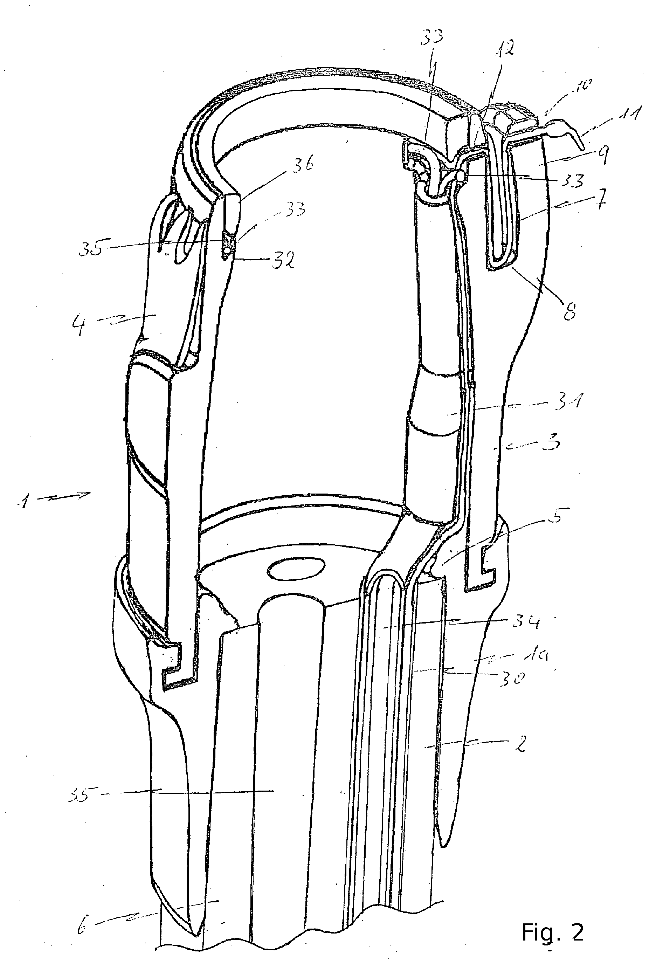

In der Fig. 2 ist eine (Endoskop-) kappe 1 gemäß einem ersten bevorzugten Ausführungsbeispiel der Erfindung am distalen Ende eines Endoskops, Trokars oder dergleichen schaftartigen Einführmittel angeordnet, welches in ein Hohlorgan eines menschlichen oder tierischen Körpers beispielsweise den Kolon oder den Magen einführbar ist.

Die Endoskopkappe 1 gemäß der Erfindung hat einen Aufsteckabschnitt 1a (vorzugsweise eine Silikontülle), der im montierten Zustand einen distalen Endoskopkopf oder Endabschnitt des Einführmittels 2 umgibt, welcher wahlweise mit endoskopspezifischen Funktionen, beispielsweise Beleuchtung, Optik, Spüleinrichtung, Arbeitskanalmündung, etc. ausgestattet ist, die von einer am proximalen Ende des Endoskops befindlichen Handhabe individuell bedienbar sind. Alternativ kann die Einführhilfe auch ein einfacher Hohlschaft ohne Zusatzfunktionen sein, der starr oder flexibel ausgeführt ist.

Die Endoskopkappe 1 ist im Axialabstand zum Aufsteckabschnitt 1a mantelseitig zu oder mit einer Spreizhülse (Spreizhülsenabschnitt) 3 ausgeformt, die im vorliegenden Ausführungsbeispiel mit dem Aufsteckabschnitt 1a formschlüssig verbunden (verclipt) ist. Sie kann aber auch einstückig mit dem Aufsteckabschnitt 1 a verbunden oder an diesem angeklebt oder angeschweißt sein. Auf die Spreizhülse 3 ist ein Gewebeclip 4 aufschiebbar, wie er vorstehend, anhand der Fig. 1 näher beschrieben wurde und somit ebenfalls zum Erfindungsgegenstand zählt. Die Spreizhülse 3 steht über die distale Stirnseite des Endoskopkopfs 2 axial vor und bildet somit einen an ihrer vorderen Kante radial nach Außen abgerundeten becher- oder napfförmigen Hülsenabschnitt.

Zur exakten axialen Positionierung der Endoskopkappe 1 hat der Steckabschnitt 1a eine radial innere Umfangskante 5 (Rückhalter oder Stopper), die sich an die Stirnseite des Endoskopkopfs 2 andrückt und somit ein Verschieben der Endoskopkappe 1 längs des Endoskops in Richtung zu dessen proximalen Ende unterbindet.

Im vorliegenden ersten Ausführungsbeispiel ist die Endoskopkappe 1 als ein zum Endoskopkopf 2 separates Bauteil gefertigt, welches auf ein bereits vorhandenes Gehäuse des Endoskopkopfs 2 (Schaftende) wahlweise mit den entsprechenden Funktionen auf- oder angesetzt ist und daher grundsätzlich auch als Nachrüstsatz
handelsüblicher Endoskope oder dergleichen schaftartiger Einführmittel geeignet ist. Alternativ hierzu kann die Endoskopkappe 1 jedoch gleichzeitig auch das Gehäuse für den Endoskopkopf 2 selbst bilden und demzufolge als Bestandteil des Endoskops fest und dichtend mit einem Endoskopschaft 6 verbunden sein, welcher in der Fig. 2 lediglich andeutungsweise dargestellt ist.

Die Spreizhülse 3 gemäß der Erfindung weist in jedem Fall eine von ihrer distalen Stirnseite aus in Axialrichtung eingebrachte Stirnnut 7 in der mantelseitigen Kappen- bzw. Hülsenwand auf, welche sich vorzugsweise als teilkreis- bzw. sichelförmiger (Umfangs-) Schlitz an der distalen Stirnseite der Spreizhülse 3 öffnet und deren Nutengrund an einer axial hinteren Stelle, vorzugsweise etwa in einem axial mittleren Abschnitt der Spreizhülse 3 einen Anschlag 8 bildet. Der Radius der Stirnnut 7 ist jedoch größer gewählt als der Außenradius der Spreizhülse 3, sodass die Hülsenwand bei der Ausbildung der Stirnnut 7 zwei in Umfangsrichtung entsprechend beabstandete Schlitze erhält. Durch Ausbilden dieser Stirnnutschlitze wird die Kappenmantelwand in diesem Bereich somit längsgespalten, wodurch an der Außenseite der Kappenwand eine Art Lasche oder Zunge 9 entsteht, welche die radial äußere Nutenwand definiert.

Eine weitere Variante der Bereitstellung einer Stirnnut gemäß vorstehender Definition ist die zusätzliche Anordnung einer vorzugsweise in Axialrichtung gekrümmten Lasche oder Zunge, wie dies insbesondere in der Fig. 2 gezeigt ist, deren Wurzel einstückig mit der Kappe ausgebildet ist und die sich unter Ausbildung der Nut im Radialabstand zur Kappenmantelwand axial in Richtung der Spreizhülse erstreckt. In diesem Fall wird also die Mantelwand nicht gespalten (wie vorstehend beschrieben), sondern es wird ein zusätzliches Bauteil in Form der Lasche über die Mantelwand der Kappe geführt. Diese Lasche kann derart schmal dimensioniert sein, dass sie im Querschnitt geradlinig (ohne Radius) bleibt, d.h. sie muss nicht notwendiger Weise dem Kappenumfang folgen. Darüber hinaus kann die Grundrissform der Lasche dann weitestgehend beliebig gestaltet werden, d.h. sie kann sich in Richtung Laschenwurzel (Übergangsbereich zwischen Lasche und Kappe) verdicken und/oder verbreitern, um so eine größere Steifigkeit zu erhalten. Auch die Laschenwurzel selbst kann nach statischen Gesichtspunkten zur Erreichung einer möglichst hohen Steifigkeit frei dimensioniert und gestaltet werden.

Unabhängig davon, nach welcher Fertigungsvariante die Lasche 9 letztlich gebildet wird, erstreckt sie sich erfindungsgemäß vom, den Anschlag 8 darstellenden Nutengrund in Richtung zur distalen Stirnseite der Kappe 1 bzw. der Spreizhülse 3, wobei deren abgerundete freie Vorderkante gegenüber der distalen Vorderkante der Spreizhülse 3 axial geringfügig zurückgesetzt ist.

Wie in der Fig. 2 zumindest angedeutet ist, erstreckt sich die Stirnnut 7 nicht exakt parallel zur Kappenmittelachse sondern ist in Richtung distaler Stirnseite zur Mittelachse hin geneigt, sodass ein darin eingesteckter Clip 4 leichter nach vorn abgleiten kann. Darüber hinaus ist die Nut 7 nicht geradlinig sondern deren Nutenwände, zumindest die äußere Nutenwand, sind in Axialrichtung leicht gekrümmt, derart, dass sich die Nut 7, zumindest die Lasche 9 in ihrem axialen Mittenabschnitt radial nach Außen wölbt. Auf diese Weise wird das Zusammenklappverhalten eines abgleitenden Gewebeclip 4 mit der Konstruktion gemäß der Fig. 1 bereits in diesem Zustand geometrisch zugelassen bzw. erleichtert.

In einem axial vorderen Endabschnitt der Lasche 9 ist diese mit einer radialen äußeren Durchgangsbohrung 10 versehen, durch die ein Faden 11, Kabel oder Gewebe vom Nuteninneren in Richtung nach Außen der Kappe 1 geführt und darin fixiert ist. Vorzugsweise ist das eine Fadenende zu diesem Zweck an der Laschenaußenseite verknotet, sodass ein Zurückziehen des Fadens 11 durch die radiale Durchgangsbohrung 10 verhindert wird. Des Weiteren ist die Endoskopkappe 1 an einer Stelle im Wesentlichen radial gegenüberliegend zur vorstehend genannten Durchgangsbohrung 10, d.h. im distalen Endbereich der axial überstehenden Spreizhülse 3 mit einer radialen inneren Durchgangsbohrung 12 versehen, durch welche der Faden 11 vom Nuteninneren nach Innen in die Spreizhülse 3 geführt ist.

Wie insbesondere aus der Fig. 2 zu entnehmen ist, befindet sich die innere Durchgangsbohrung 12 axial vor der distalen Stirnseite des Endoskopkopfs 2, sodass der Faden 11 aus der inneren Durchgangsbohrung 12 kommend in einen an der Schaft-Stirnseite sich öffnenden Funktionskanal oder den Arbeitskanal 30 des Endoskopschafts 6 einfädelbar ist, ohne dass dieser eine lange freie Wegstrecke zurücklegen muss. Dennoch befindet sich innerhalb der Spreizhülse 3 eine Art Verblendung oder Schacht 31, die sich längs der Hülse 3 erstreckt und den Faden 11 zwischen der inneren Durchgangsbohrung 12 und distalem Ende des Endoskopschafts 6 abdeckt.

Der Innenumfang (Innenwand) der Spreizhülse 3 ist an ihrem distalen Endabschnitt über eine vorbestimmte axiale Strecke von ca. 2 bis 6 mm und vorzugsweise 2 bis 3.5 mm ausgedreht, wodurch sich ein Innenabsatz 32 (in Vorschubrichtung gesehen) unmittelbar vor der inneren Durchgangsbohrung 12 ausbildet. An diesem Innenabsatz 32 liegt eine Kabelschlinge 33 an, die über ein durch den Schacht 31 sowie den Endoskopschaft 6 geführtes Stromkabel 34 betätigbar und bestrombar ist. Des Weiteren ist in die innere Ausdrehung 35 ein vorzugsweise ringförmiges Distanzstück 36 eingesetzt, welches die Kabelschlinge 33 gegen den Innenabsatz 32 drückt, derart, dass der Faden 11 ungehindert aus der inneren Durchgangsbohrung 12 gezogen werden kann.

An dieser Stelle sei darauf hingewiesen, dass gemäß dem ersten bevorzugten Ausführungsbeispiel der Erfindung sowohl der Faden 11 als auch das Stromkabel 34 in hierfür vorgesehene Funktionskanäle 30 im Endoskopschaft 6 geführt sind. Sie können aber auch in einem Arbeitskanal 35 verlegt sein, durch den normaler Weise medizinische Instrumente eingeführt werden.

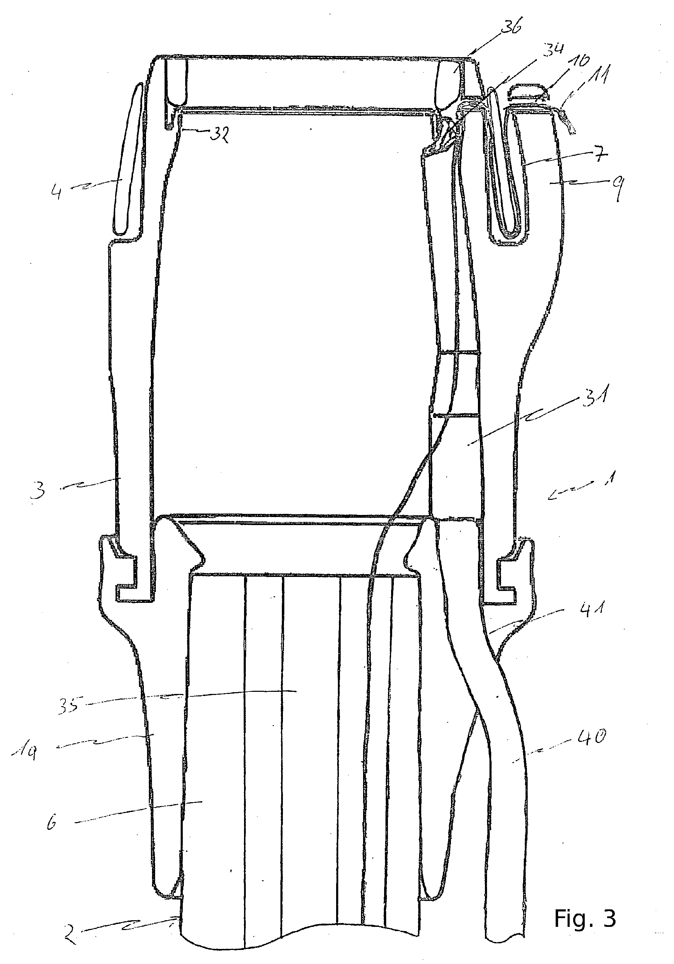

Alternativ hierzu ist es aber auch möglich, gemäß der Fig. 3 lediglich den Faden 11 innerhalb des Endoskopschafts 6 zu verlegen und das Stromkabel 34 über einen äußeren Kabelkanal 40 zu führen. In diesem Fall ist gemäß dem zweiten Ausführungsbeispiel der Erfindung die (Endoskop-) kappe 1 im Bereich ihres Aufsteckabschnitts 1a mit einer Axialbohrung 41 versehen, in die der Kabelkanal 40 eingesteckt ist und durch die das Stromkabel 34 hindurchgeführt ist.

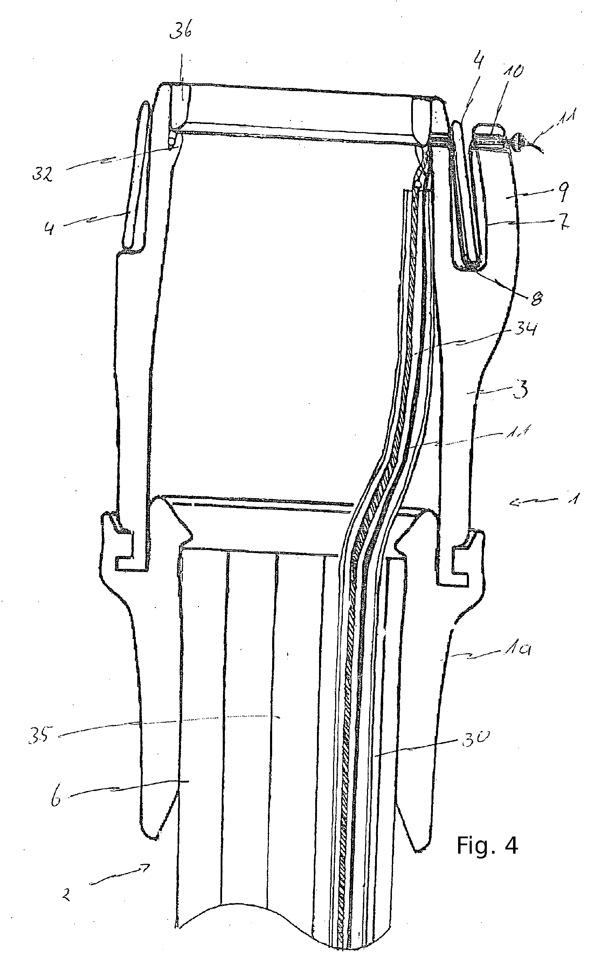

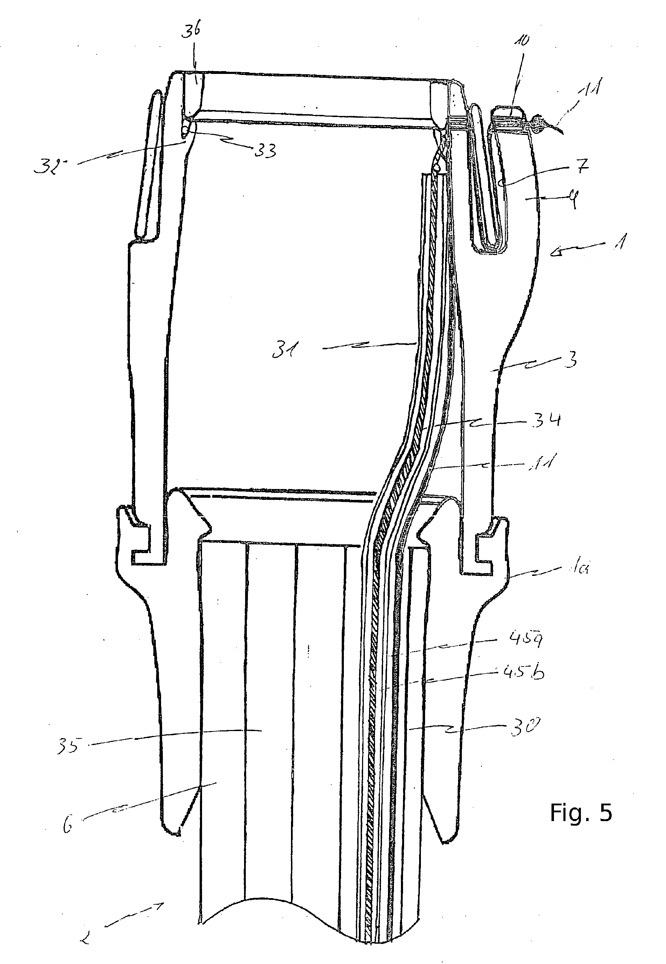

Auch ist es möglich, gemäß dem in Fig. 4 dargestellten dritten bevorzugten Ausführungsbeispiel der Erfindung das Stromkabel 34 wie auch den Faden 11 in dem gleichen Funktionskanal 30 innerhalb des Endoskopschafts 6 frei zu führen. Eine Alternative hierzu gemäß der Fig. 5 sieht vor, das Stromkabel 34 sowie den Faden 11 in getrennten Schläuchen 45a, 45b zu führen, die wiederum innerhalb eines Funktionskanals 30 des Endoskopschafts 6 verlegt sind und somit den Faden 11 und das Stromkabel 34 voneinander trennen.

Gemäß der Fig. 2 ist das Distanzstück 36 als ein in die Spreizhülse 3 eingesetztes separates Bauteil gefertigt. Alternativ hierzu kann aber auch die Spreizhülse 33 an ihrer Innenseite mit einer Umfangsnut im Bereich des gezeigten Innenabsatzes 32 ausgebildet sein, in die die Kabelschlinge 33 eingedrückt werden kann. Auch könnte die Kabelschlinge 33 in diesem Bereich an die Innenwand der Spreizhülse 3 lösbar angeklebt oder angeheftet sein. Schließlich ist es auch möglich, die innere Umfangsnut in Axialrichtung abzuschrägen, um auf diese Weise ein Aufnahmeraum für die Kabelschlinge 33 zu schaffen. Vorzugsweise kann das Distanzstück aus einem flexiblen oder elastischen Material wie Silikon gefertigt sein, das ein Herausziehen der Kabelschlinge 33 erleichtert.

Entscheidend ist bei allen genannten Varianten, dass die Kabelschlinge 33 in einem vorbestimmten axialen Abstand von vorzugsweise 2 bis 6 mm zur distalen Stirnseite der Kappe 1 gehalten wird, solange, bis diese durch Zug über das Stromkabel 34 ausgelöst, d.h. von der Innenwand der Spreizhülse 3 abgezogen wird.

Die Arbeitsweise der erfindungsgemäßen Endoskopkappe 1 mit der Halte- und Abziehfunktion für den Gewebeclip 4 sowie der Schneidevorrichtung wird nachfolgend näher beschrieben.

Um einen Gewebeclip 4 beispielsweise gemäß der Fig. 1 an seine vorbestimmte Position zu bringen, muss dieser zuerst auf die Spreizhülse 3 der Endoskopkappe 1 aufgezogen werden. Hierfür werden Unter- und Oberkiefer des Gewebeclips 4 von Hand aufgeklappt, sodass der Clip 4 an der gerundeten Vorderkante der Spreizhülse 3 angesetzt und über diese geschoben werden kann. Dabei dringt die Hinterkante des Gewebeclips 4 in die Stirnnut 7 der Endoskopkappe 1 ein und zieht dabei den Faden 11 aus dem Funktions- oder Arbeitskanal 30 des Endoskopschafts 6 heraus.

Schließlich kommt die Schiebebewegung des Clips 4 mit dessen in Anlage kommen am Nutengrund 8 zum Stillstand, wobei der Clip 4 sowie der mitgenommene Faden 11 die in Fig. 2 gezeigte Raumlage einnehmen. D.h. in dieser Lage ist der Clip 4 vollständig auf die Endoskopkappe 1 aufgezogen und kann so über das Endoskop 2 in ein Hohlorgan eingebracht werden. Der Faden 11 umgreift dabei die Hinterkante des Clips 4 und erhält somit eine U-Form in Fadenlängsrichtung gesehen.

Sobald die erfindungsgemäße Resektionsvorrichtung an eine erkrankte Stelle innerhalb eines Hohlorgans gelangt ist, wird die Spreizhülse 3 gegen die Organwand gedrückt und die Wand durch Unterdruck innerhalb der Spreizhülse 3 und/oder mittels einer über den Endoskopschaft 6 (dessen Arbeitskanal 35) eingeführten Zange oder dergleichen Greifinstrument in die Spreizhülse 3 eingezogen. Soll der Clip 4 nunmehr abgestreift werden, wird der Faden 11, welcher durch den Schaftkanal 30 bis zum proximalen Ende des Endoskops 2 geführt ist, gezogen, wobei sich der die Stirnnut 7 in Radialrichtung durchquerende Fadenabschnitt verkürzt. Da der Faden 11 in der äußeren Durchgangsbohrung 10 fixiert ist, übt dieser mit einer entsprechenden Übersetzung nach dem Flaschenzugprinzip auf den Clip 4 eine Kraft in Axialrichtung aus, wodurch der Clip 4 in Richtung distales Ende der Endoskopkappe 1 verschoben wird. Durch die äußere Abrundung der vorderen Spreizhülsenkante sowie die weiche, d.h. gewölbte Formgebung der Stirnnut 7 (insbesondere der Lasche 9) wird ein Abgleiten des Clips 4 über die Vorderkante der Spreizhülse 3 erleichtert und die über den Faden 11 aufzubringende maximale Verschiebekraft weiter reduziert. Sobald die Hinterkante des Clips 4 aus der Stirnnut 7 getreten ist und daher nicht mehr von der Lasche 9 gehalten werden kann, bewirkt die im Clip 4 gespeicherte Vorspannkraft ein Abspringen des Clips 4 von der Spreizhülse 3, wodurch die Organwand im Bereich unmittelbar vor der Spreizhülse 3 abgeklemmt wird.

Jetzt kann die Schneideeinrichtung betätigt werden. Hierzu wird das Stromkabel 34 gezogen, wodurch sich die Kabelschlinge 33 von der Innenwand der Spreizhülse 3 ablöst und die eingezogene Organwand ca. 2 bis 6 mm vor dem Gewebeclip 4 einschnürt. Durch Bestromen der Schlinge 33 mit Hochfrequenzstrom wird die eingezogene Wand abgeschert. Damit ist die Organwandresektion beendet und das Endoskop 2 kann zusammen mit dem abgescherten Wandstück aus dem Hohlorgan genommen werden, wobei der Clip 4 die Wunde verschließt.

An dieser Stelle sei noch darauf hingewiesen, dass die als Kabelschlinge 33 definierte Schneidevorrichtung nur eine Variante darstellt und durch eine andere Art einer Schneidevorrichtung, beispielsweise eine Klinge oder eine rosettenartig schließende Schere ersetzt werden kann, was jedoch nicht Teil der vorliegenden Erfindung ist.

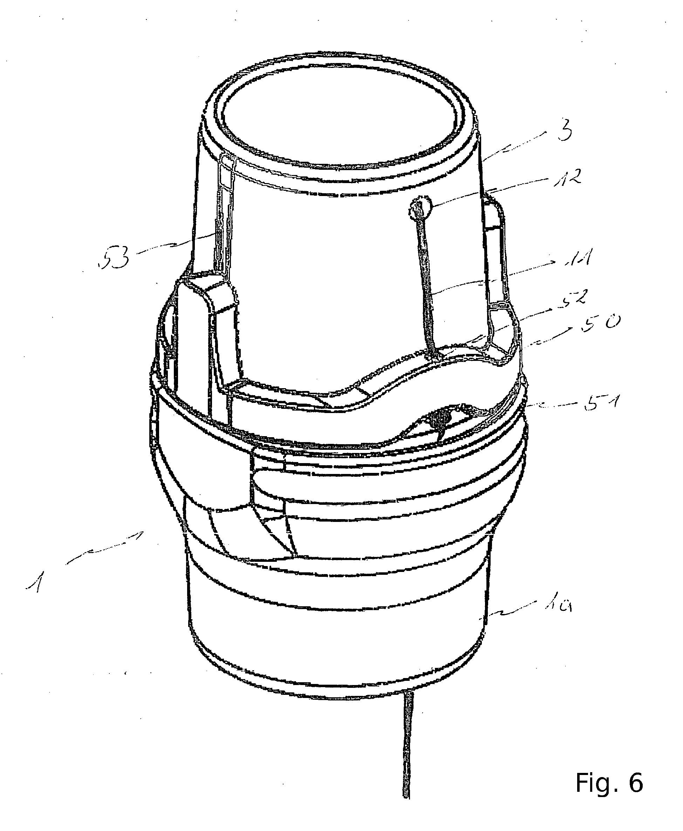

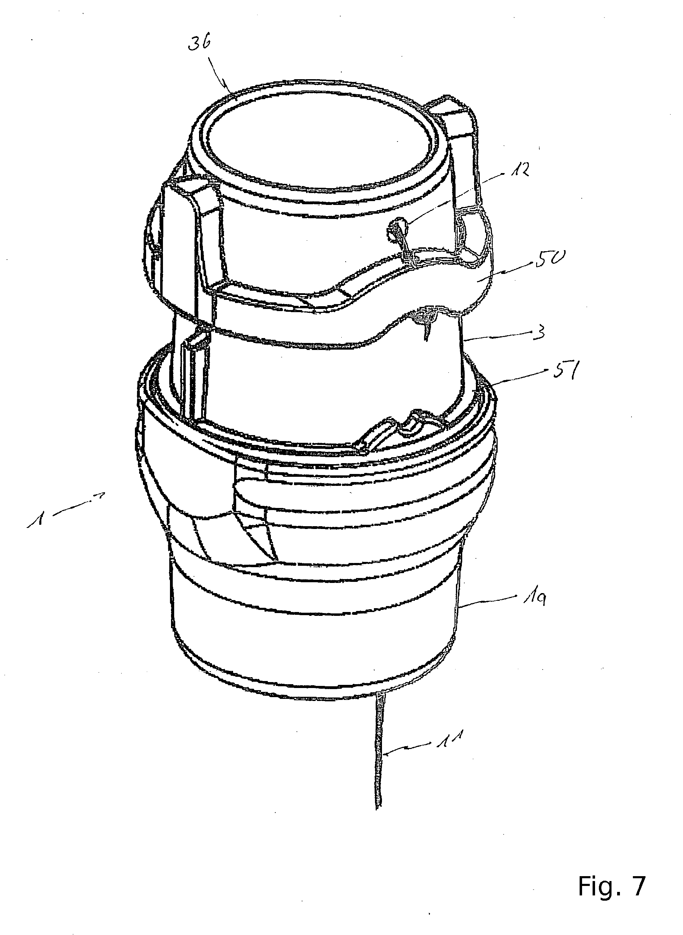

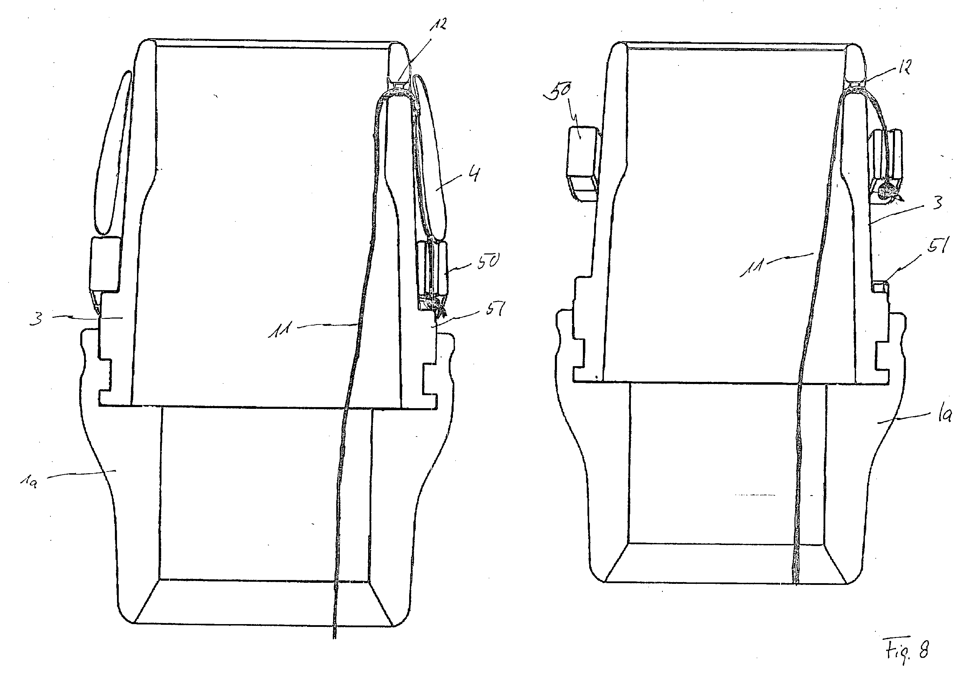

Die Fig. 6 bis 8 zeigen ein weiteres Ausführungsbeispiel der Erfindung, wobei im Nachfolgenden lediglich auf die zu den vorstehenden Ausführungsbeispielen unterschiedlichen Merkmale eingegangen werden soll.

Wie aus der Fig. 6 zu entnehmen ist, besteht die Abziehvorrichtung des fünften bevorzugten Ausführungsbeispiels der Erfindung aus einem Abziehring 50, der auf die Spreizhülse 3 gezogen ist und sich gegen einen äußeren Wellenabsatz 51 im Mittenbereich der Endoskopkappe 1 anlegt. Alternativ hierzu kann sich der Abziehring 50 aber auch gegen den Nutengrund einer Stirnnut gemäß einem der vorstehenden Ausführungsbeispiele axial anlegen.

Im fünften Ausführungsbeispiel ist keine Lasche oder Stirnnut vorgesehen. Statt dessen ist der durch die innere Durchgangsbohrung 12 durchgeführte Faden 11 unmittelbar am Abziehring 50 befestigt, indem in diesen eine Durchgangsbohrung 52 eingebracht ist, durch die der Faden 11 geführt und darin fixiert ist. Des Weiteren ist am Außenumfang der Spreizhülse 3 ein axialer Steg 53 oder eine Axialnut ausgeformt, der in eine axiale Innennut des Abziehrings 50 oder ein axialer Innensteg greift und eine Axialführung für den Abziehring 50 bildet. Im Übrigen ist der Abziehring 50 hinsichtlich seiner Form dem Gewebeclip (in der Fig. 6 nicht dargestellt) angeglichen, sodass dieser im Wesentlichen passgenau an den Ring 50 angelegt werden kann.

Die Funktionsweise der Resektionsvorrichtung des fünften Ausführungsbeispiels der Erfindung lässt sich anhand der Fig. 6 bis 8 wie Folgt beschreiben.

Sobald eine erkrankte Organwand in die Spreizhülse 3 per Unterdruck und/oder Zangeninstrument (Greifinstrument) eingezogen ist, wird der Gewebeclip abgezogen. Hierzu muss der Faden 11 längs des Endoskopschafts gezogen werden, wodurch sich der Abziehring 50 nach vorn in Richtung zur distalen Stirnkante der Spreizhülse 3 bewegt. Dabei wird auch der Clip nach vorn verschoben, bis dieser durch dessen Federvorspannung über die distale Stirnkante der Spreizhülse 3 abspringt und das Organgewebe zwischen seinen Kiefern einklemmt. Hierauf kann das eingezogene Gewebe mittels der Schneidevorrichtung innerhalb der Spreizhülse 3 abgeschert werden.

## Patentansprüche

1. Resektionsvorrichtung mit einer becher- oder napfförmige Kappe (1) für ein schaftartiges Einführmittel (2), die am distalen Ende des schaftartigen Einführmittels (2) befestigt oder daran ausgebildet ist und die einen Spreizhülsenabschnitt (3) hat, auf den ein federelastisch vorgespannter Gewebeclip (4) aufgesteckt ist, der mittels einer Auslöse- oder Abziehvorrichtung (11, 50) über die distale Stirnkante der Kappe (1) abziehbar ist, **dadurch gekennzeichnet, dass** im Inneren des Spreizhülsenabschnitts (3) eine Schneidevorrichtung (33) in Form einer Kabelschlinge angeordnet ist, die an der Innenwand des Spreizhülsenabschnitts (3) in einem vorbestimmten axialen Abstand zur distalen Stirnkante der Kappe (1) gehalten ist, wofür an der Innenwand des Spreizhülsenabschnitts (3) ein Distanzstück (36) vorzugsweise in Form eines Rings angeordnet oder einstückig mit der Kappe (1) ausgebildet ist, derart, dass sich am distalen Endbereich des Spreizhülsenabschnitts (3) eine innere Umfangsnut ausbildet, die entsprechend dem Distanzstück (36) axial von der distalen Stirnseite des Spreizhülsenabschnitts (3) beabstandet ist und eine Klemmfläche für die Kabelschlinge bildet.

2. Resektionsvorrichtung nach Anspruch 1, **dadurch gekennzeichnet, dass** die Innenwand des Spreizhülsenabschnitts (3) ausgehend von der distalen Stirnkante eine radiale Ausdrehung (35) aufweist, in die das Distanzstück (36) eingesetzt ist, wodurch zwischen dem Distanzstück (36) und einem durch die Ausdrehung (35) gebildeten Innenabsatz (32) die innere Umfangsnut ausgeformt ist.

3. Resektionsvorrichtung nach Anspruch 1, **dadurch gekennzeichnet, dass** an der Innenwand des Distanzhülsenabschnitts (3) in dem vorbestimmten Axialabstand zur distalen Stirnkante der Kappe (1) die innere Umfangsnut ausgedreht ist, wodurch das Distanzstück (36) einstückig mit der Kappe (1) ausgebildet wird.

4. Resektionsvorrichtung nach einem der vorstehenden Ansprüche, **dadurch gekennzeichnet, dass** der vorbestimmte Axialabstand 2 bis 6 mm, vorzugsweise 2 bis 3.5 mm beträgt.

5. Resektionsvorrichtung nach einem der vorstehenden Ansprüche, **dadurch gekennzeichnet, dass** die Auslöse- oder Abziehvorrichtung (11) ein Faden, Kabel oder Gewebe ist, der durch das Innere der Kappe (1) hindurch geführt und entweder um die distale Stirnkante herum oder durch eine radiale Durchgangsbohrung (12) unmittelbar vor der distalen Stirnkante in dem Spreizhülsenabschnitt (3) nach Außen geleitet ist, um den Gewebeclip (4) in Richtung zur distalen Stirnkante zu ziehen.

6. Resektionsvorrichtung nach Anspruch 5, **dadurch gekennzeichnet, dass** eine in Umfangsrichtung des Spreizhülsenabschnitts (3) beidseitig geöffnete Stirnnut (7) am Außenumfang des Spreizhülsenabschnitts (3) zur axialen Aufnahme des Gewebeclips (4) ausgebildet ist, durch die der Faden (11), Kabel oder Gewebe in Radialrichtung gezogen und an einem freien Ende an der Kappe (1) fixiert ist.

7. Resektionsvorrichtung nach Anspruch 5, **dadurch gekennzeichnet, dass** der Faden (11), Kabel oder Gewebe an seinem einen freien Ende an einem Abstreifring (50) befestigt ist, der auf dem Spreizhülsenabschnitt (3) hinter dem aufgezogenen Clip (4) axialverschieblich sitzt und bei Ziehen des Fadens (11) nach vorn in Richtung zur distalen Stirnseite der Kappe (1) verschiebbar ist und dabei den Clip (4) vor sich weg drückt.

8. Resektionsvorrichtung nach einem der vorstehenden Ansprüche, **dadurch gekennzeichnet, dass** das Einführmittel (2) ein Endoskop mit einem Endoskopschaft (6) ist, in dem ein Arbeitskanal (35) zum Zuführen eines medizinischen Instruments beispielsweise einer Zange ausgebildet ist.

9. Resektionsvorrichtung nach Anspruch 8, **dadurch gekennzeichnet, dass** das Stromkabel (34) und der Fader (11), Kabel oder Gewebe innerhalb des Endoskopschafts im Arbeitskanal (35) oder einem hierzu parallelen Funktionskanal (30) geführt sind.

10. Resektionsvorrichtung nach Anspruch 8, **dadurch gekennzeichnet, dass** der Faden (11), Kabel oder Gewebe innerhalb des Endoskopschafts (6) und das Stromkabel (34) außerhalb des Endoskopschafts in einem externen Schlauch (40) geführt sind.

## Claims

1. A resection device comprising a cup-shaped cap (1) for a shaft-type inserting means (2) which is fixed at the distal end of the shaft-type inserting means (2) or formed at the same and which includes an expanding sleeve portion (3) to which a spring-biased tissue clip (4) is attached which is removable by means of a releasing or withdrawing device (11, 50) over the distal front edge of the cap (1), **characterized in that** inside the expanding sleeve portion (3) a cutting device (33) in the form of a cable loop is arranged, which is held at the inner wall of the expanding sleeve portion (3) at a predetermined axial distance from the distal front edge of the cap (1), for which at the inner wall of the expanding sleeve portion (3), a spacer (36) is arranged preferably in the form of a ring or is formed integrally with the cap (1), such that the expanding sleeve portion (3) forms at its distal end portion an inner circumferential groove which is, corresponding to the spacer (36), axially spaced from the distal end face of the expanding sleeve portion (3) and forms a clamping surface for the cable loop.

2. The resection device according to claim 1, **characterized in that** starting out from the distal front edge the inner wall of the expanding sleeve portion (3) exhibits a radial bore (35) into which the spacer (36) is inserted, whereby the inner circumferential groove is shaped between the spacer (36) and an inner shoulder (32) formed by the bore (35).

3. The resection device according to claim 1, **characterized in that** at the inner wall of the spacer portion (3) the inner circumferential groove is bored at the predetermined axial distance from the distal front edge of the cap (1), thereby the spacer (36) being formed integrally with the cap (1).

4. The resection device according to any one of the preceding claims, **characterized in that** the predetermined axial distance is 2 to 6 mm, preferably 2 to 3.5 mm.

5. The resection device according to any one of the preceding claims, **characterized in that** the releasing or withdrawing device (11) is a thread, cable or tissue which is guided through the interior of the cap (1) and is guided either around the distal front edge or through a radial through bore (12) directly ahead of the distal front edge in the expanding sleeve portion (3) to the outside so as to pull the tissue clip (4) in the direction of the distal front edge.

6. The resection device according to claim 5, **characterized in that** a front groove (7) opened on both sides in the circumferential direction of the expanding sleeve portion (3) is formed at the outer circumference of the expanding sleeve portion (3) for axially receiving the tissue clip (4) through which front groove the thread (11), cable or tissue is pulled in radial direction and is fixed at a free end of the cap (1).

7. The resection device according to claim 5, **characterized in that** the thread (11), cable or tissue is fixed at its one free end to a strip ring (50) which is seated to be axially movable on the expanding sleeve portion (3) behind the slipped-on clip (4) and, when pulling the thread (11), is movable forward in the direction of the distal end face of the cap (1) while pressing away the clip (4) ahead.

8. The resection device according to any one of the preceding claims, **characterized in that** the inserting means (2) is an endoscope having an endoscope shaft (6) in which a working channel (35) for feeding a medical instrument, for instance forceps, is formed.

9. The resection device according to claim 8, **characterized in that** the electric lead (34) and the thread (11), cable or tissue are guided inside the endoscope shaft in the working channel (35) or a function channel (30) parallel thereto.

10. The resection device according to claim 8, **characterized in that** the thread (11), cable or tissue is guided inside the endoscope shaft (6) and the electric lead (34) is guided outside the endoscope shaft in an external tube (40).

## Revendications

1. Dispositif de résection comprenant un capuchon (1) en forme de pot ou de cuvette destiné à un moyen d'introduction (2) de type tige, qui est fixé au niveau de l'extrémité distale du moyen d'introduction (2) de type tige ou est formé au niveau de cette dernière et qui comporte une section de manchon à expansion (3), sur laquelle est enfiché un clip tissulaire (4) précontraint de manière élastique, qui peut être retiré par l'arête frontale distale du capuchon (1) au moyen d'un dispositif de déclenchement ou de retrait (11, 50), **caractérisé en ce qu'**un dispositif de coupe (33) se présentant sous la forme d'une élingue de câble est disposé à l'intérieur de la section de manchon à extension (3), lequel dispositif de coupe est maintenu au niveau de la paroi intérieure de la section de manchon à expansion (3) à une distance axiale prédéterminée par rapport à l'arête frontale distale du capuchon (1), une pièce d'écartement (36) se présentant de préférence sous la forme d'un anneau étant disposée à cet effet au niveau de la paroi intérieure de la section de manchon à expansion (3) ou étant réalisée d'un seul tenant avec le capuchon (1) de manière à former, au niveau de la zone d'extrémité distale de la section de manchon à expansion (3), une rainure intérieure périphérique, qui est espacée axialement du côté frontal distal de la section de manchon à expansion (3) de manière à correspondre à la pièce d'écartement (36) et qui forme une surface de serrage pour l'élingue de câble.

2. Dispositif de résection selon la revendication 1, **caractérisé en ce que** la paroi intérieure de la section de manchon à expansion (3) présente un alésage (35) radial partant de l'arête frontale distale, dans lequel alésage est insérée la pièce d'écartement (36) ce qui permet de former la rainure intérieure périphérique entre la pièce d'écartement (36) et un décrochement intérieur (32) formé par l'alésage (35).

3. Dispositif de résection selon la revendication 1, **caractérisé en ce que** la rainure périphérique intérieure est alésée au tour au niveau de la paroi intérieure de la section de manchon d'écartement (3), à la distance axiale prédéterminée par rapport à l'arête frontale distale du capuchon (1), ce qui permet de réaliser la pièce d'écartement (36) d'un seul tenant avec le capuchon (1).

4. Dispositif de résection selon l'une quelconque des revendications précédentes, **caractérisé en ce que** la distance axiale prédéterminée est comprise entre 2 et 6 mm, de préférence entre 2 et 3,5 mm.

5. Dispositif de résection selon l'une quelconque des revendications précédentes, **caractérisé en ce que** le dispositif de déclenchement ou de retrait (11) est un fil, un câble ou un tissu, qui est guidé au travers de l'intérieur du capuchon (1) et qui est conduit vers l'extérieur soit tout autour de l'arête frontale distale ou en passant par un alésage de passage (12) radial directement avant l'arête frontale distale, dans la section de manchon à expansion (3) pour tirer le clip tissulaire (4) en direction de l'arête frontale distale.

6. Dispositif de résection selon la revendication 5, **caractérisé en ce qu'**une rainure frontale (7) ouverte de part et d'autre dans la direction périphérique de la section de manchon à expansion (3) est réalisée au niveau de la périphérie extérieure de la section de manchon à expansion (3) aux fins du logement axial du clip tissulaire (4), par laquelle le fil (11), le câble ou le tissu sont tirés dans la direction radiale et sont fixés au niveau d'une extrémité libre au niveau du capuchon (1).

7. Dispositif de résection selon la revendication 5, **caractérisé en ce que** le fil (11), le câble ou le tissu sont fixés au niveau d'un anneau racleur (50), au niveau de l'une des extrémités libres de ce dernier, lequel repose de manière à pouvoir être déplacé par coulissement axialement, sur la section de manchon à expansion (3), derrière le clip (4) ouvert et peut être déplacé par coulissement, lorsque le fil (11) est tiré vers l'avant, dans la direction du côté frontal distal du capuchon (1) et pousse ce faisant le clip (4) de sorte que ce dernier s'éloigne de lui.

8. Dispositif de résection selon l'une quelconque des revendications précédentes, **caractérisé en ce que** le moyen d'introduction (2) est un endoscope doté d'une tige d'endoscope (6), dans lequel est réalisé un canal de travail (35) servant à amener un instrument médical par exemple une pince.

9. Dispositif de résection selon la revendication 8, **caractérisé en ce que** le câble du courant (34) et le fil (11), le câble ou le tissu sont guidés à l'intérieur de la tige d'endoscope dans le canal de travail (35) ou dans un canal fonctionnel (30) parallèle audit canal de travail.

10. Dispositif de résection selon la revendication 8, **caractérisé en ce que** le fil (11), le câble ou le tissu sont guidés à l'intérieur de la tige d'endoscope (6) et **en ce que** le câble du courant (34) est guidé en dehors de la tige d'endoscope, dans un flexible (40) externe.
